# EUROPEAN PATENT SPECIFICATION

(11) **EP 2 620 111 B1**
(45) Date of publication and mention of the grant of the patent: **30.08.2017**
(21) Application number: 13152223.7
(22) Date of filing: 22.01.2013
(51) Int. Cl.: A61B 17/34

(54) **Echogenic medical device**
Echogene medizinische Vorrichtung
Dispositif médical échogénique

(30) Priority: 25.01.2012 US 201261590495 P
(43) Date of publication of application: 31.07.2013
(73) Proprietor: Cook Medical Technologies LLC, Bloomington, IN 47404 (US)
(72) Inventor: Fischer, Frank J. Jr., Bloomington, IN 47401 (US)
(74) Representative: Georgiou, Sarah Caroline

(56) References cited:
- WO-A1-2010/128150
- US-A- 5 766 135
- US-A1- 2003 040 756
- US-A1- 2008 097 213
- US-A1- 2009 182 192
- US-A1- 2011 160 592
- US-A1- 2011 190 660

## Description

### BACKGROUND OF THE INVENTION

Technical Field. The present invention relates to a medical device constructed for visualization within the body of a patient under medical imaging. More particularly, the invention relates to an echogenic medical device configured such that the location and rotational orientation of the device in the body of a patient may be observed in real time under ultrasound visualization.

Background Information. The ability to monitor the location and orientation of surgical instrumentation within intraluminal and extraluminal regions of the body of a patient has attained increased importance in recent years. Instruments formed of fluoroscopic and radiopaque materials are widely used to create visible regions within the body. Fluoroscopy is a technique in which an x-ray beam is transmitted through a patient to generate images of the target structure that can be displayed in a monitor. It can also be used to observe the position of instruments during diagnostic procedures. However, the use of x-ray exposes the patient to potentially harmful radiation. Additionally, health care workers must typically transport the patient to a specially-equipped radiology facility to obtain the x-ray, thereby increasing the cost and complexity of the procedure. Further, the images obtained via fluoroscopy may not achieve sufficient clarity to provide the desired level of detail to the medical professional.

Conventional endoscopy offers visualization of the immediate regions within which the endoscope is positioned by way of a video camera attached at the distal end of the endoscope. However, the video camera provides a field of view limited to only the immediate region. Surgical instrumentation within the immediate region that is obstructed by body features or by other instrumentation cannot be visualized. Similarly, instrumentation outside of the immediate region, such as outside the lumen in which the endoscope has been positioned, cannot be visualized with the endoscopic video camera.

Ultrasound imaging is another option that has been used to monitor the placement of medical instrumentation. Ultrasound imaging utilizes high frequency sound waves to create an image of living tissue. As ultrasound waves are emitted, the waves are reflected upon encountering a surface change. The reflected waves are captured to create an image, which image is displayed on a monitor in real time. Ultrasound imaging allows for monitoring of the medical devices in extraluminal regions, as well as in intraluminal regions. Such monitoring is readily used in modern medicine to guide a medical device to a target site, while at the same time minimizing the possibility of inadvertent injury to adjacent tissue resulting from a misplaced device.

Ultrasound visualization has additional favorable characteristics in that it can be performed at the bedside, and it eliminates exposure of the patient to hazardous radiation. Although ultrasound visualization provides benefits not available with other medical techniques, there are some shortcomings associated with this technique. For example, the device to be observed under ultrasound may not be easily visible at certain angles relative to the ultrasound probe. In addition, the ultrasound image may not provide sufficient detail to enable the medical professional to determine with a high degree of confidence the particular orientation of the instrument in the viewing region, such as the degree of rotation of the device in the region.

The two part form of claim 1 is based on WO 2010/128150 A.

### SUMMARY

The present invention is defined in the appended claims.

Aspects of the present invention address at least some of the problems of the prior art. A medical device configured for insertion into the body of a patient and ultrasound-guided movement therein to an interior target site is disclosed. A shaft has a proximal portion and a distal portion, wherein the distal portion extends to a distal end. A first echogenic region at the distal portion is structured for providing a signal visible under ultrasound visualization. A second echogenic region proximal of the first echogenic region is structured and arranged for providing a signal visible under ultrasound visualization. The signal at the second echogenic region is visually distinguishable from the signal at the first echogenic region.

In a preferred embodiment the present invention provides an echogenic device that is structured such that specified features of the device can be visualized in real time when the device is positioned within the body of the patient with greater precision than available with prior art devices.

In another form a medical device configured for insertion into the body of a patient and ultrasound-guided movement therein to a target site is disclosed. A shaft has a proximal portion and a distal portion, wherein the distal portion extends to a distal end. An echogenic region at the distal portion comprises a plurality of geometric configurations disposed along the distal portion. At least some of the geometric configurations extend into a matrix of the shaft and define at least two wall angles θ₁, θ₂. Each of the wall angles is configured and positioned to enhance an echogenicity of the geometric configuration under ultrasound visualization.

In still another form thereof, an echogenic needle is disclosed. The echogenic needle includes a shaft having a proximal portion, a distal portion extending to a distal end, first and second generally opposing longitudinal sides extending along the proximal and distal portions, and a passageway extending therethrough. The distal end defines a beveled opening communicating with the passageway. The beveled opening extends between a distal tip portion disposed along the first longitudinal side and a heel portion disposed along the second longitudinal side. A first echogenic region extending circumferentially around the shaft at the distal portion is structured for providing a signal visible along substantially the entire circumference of the shaft under ultrasound visualization. A second echogenic region extending along a length of the second longitudinal side and substantially aligned with the heel portion is structured and arranged for providing a generally linear signal visible under ultrasound examination along the second longitudinal side, and substantially not visible along the first longitudinal side.

### BRIEF DESCRIPTION OF THE DRAWINGS

Embodiments of the present invention are described below, by way of example only, with reference to the accompanying drawings, in which:
Fig. 1 is a side view of the distal portion of a prior art echogenic needle;
Fig. 2 is a side view of the distal portion of the prior art echogenic needle, rotated 90 degrees from the orientation as shown in Fig. 1;
Fig. 3 is a side view of the distal portion of an echogenic needle according to an embodiment of the present invention;
Fig. 4 is a side view of the distal portion of the echogenic needle of Fig. 3, rotated 90 degrees from the orientation as shown in Fig. 3;
Fig. 5 is a side view of another example of an echogenic needle, wherein the echogenic region includes multiple echogenic shapes;
Fig. 6 is a side view of the distal portion of the echogenic needle of Fig. 5, rotated 90 degrees from the orientation as shown in Fig. 5;
Fig. 7 is a side view of still another example of an echogenic the distal portion of an echogenic needle similar to that of Fig. 5, wherein the echogenic shapes are provided in a random rotational pattern along the side of the echogenic needle;
Fig. 8 is a side view of the distal portion of the echogenic needle of Fig. 7, rotated 90 degrees from the orientation as shown in Fig. 7;
Fig. 9 is a side view of the distal portion of an echogenic needle according to yet another alternative embodiment, not according to the invention, including multiple bands of echogenic elements, wherein each band is formed of elements having a configuration that differs from the elements of another band;
Fig. 10 is a side view of the distal portion of another embodiment of an echogenic needle not according to the present invention, illustrating shaped dimples extending inwardly into the surface of the needle;
Fig. 11 is an enlarged view of one of the shaped elements of the needle of Fig. 10;
Fig. 12 is a tangential sectional view of the needle and shaped element taken along A-A of Fig. 11 and illustrating angle θ₁;
Fig. 13 is an axial sectional view of the needle and shaped element taken along B-B of Fig. 11 and showing angle θ₂;
Fig. 14 is a side view of a catheter having an echogenic ribbon applied along the top outer surface of the catheter; and
Fig. 15 is a top view of the catheter of Fig. 14.

The components in the drawings are not necessarily to scale, emphasis instead being placed upon illustrating the principles of the teachings herein.

### DESCRIPTION OF THE PREFERRED EMBODIMENTS

For purposes of promoting an understanding of the present invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

In the following discussion, the terms "proximal" and "distal" will be used to describe the opposing axial ends of the device, as well as the axial ends of various component features of the device. The term "proximal" is used in its conventional sense to refer to the end of the device (or component thereof) that is closest to the operator during use of the device. The term "distal" is used in its conventional sense to refer to the end of the device (or component thereof) that is initially inserted into the patient, or that is closest to the patient during use.

As used herein, the term "echogenic" is defined as having enhanced echogenicity. Specifically, it is used to refer to a structure, or a portion of a structure, constructed or treated in a manner to provide greater reflectivity of ultrasonic waves than the structure, or structure portion, would exhibit in the absence of such construction or treatment, and/or that is capable of providing an echogenic profile relative to surrounding tissues during use of the structure in the body of a patient.

It is known in the art that materials used for medical devices, such as a needle, sheath, catheter, cannula, stylet, etc., will reflect some ultrasonic waves. However, the term "echogenic," as used herein includes constructing or treating the device by creating, e.g., a textured, patterned, indented, angled or otherwise irregular surface including, for example, one or more dimples, divots, knurls, ridges, nubs, and the like (hereafter collectively referred to as "dimples"), each of which is known in the art to enhance echogenicity as compared to a more smooth or untreated surface for a similarly-sized/shaped object, and/or applying a material to the device capable of enhancing the echogenicity of the device when compared to a device not having the material applied thereto, and/or forming the device, or a discrete portion of the device, of a matrix suitable for enhancing echogenicity when compared to an otherwise similar device or device portion not formed of the echogenicity-enhancing matrix.

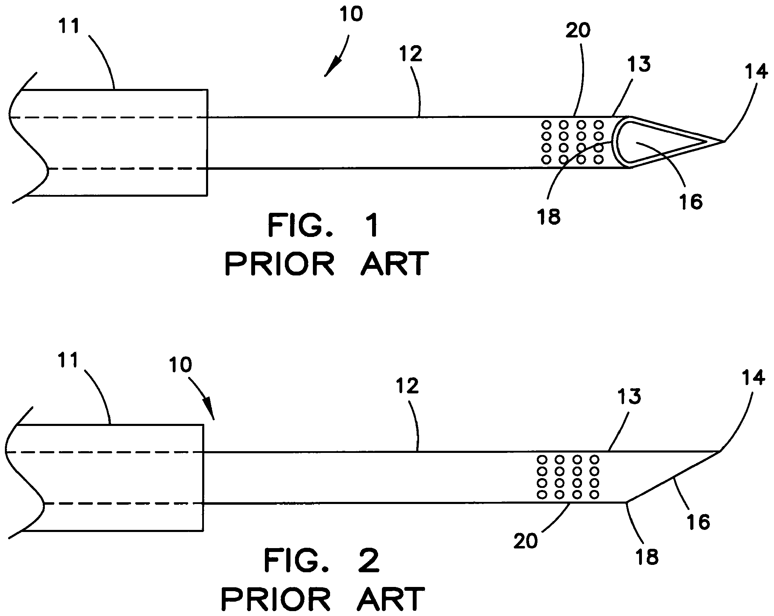

Fig. 1 illustrates a side view of the distal portion of a prior art echogenic medical device, in this case, an echogenic needle 10. Fig. 2 is a side view of the distal portion of the needle 10, rotated 90 degrees from the orientation as shown in Fig. 1. Needle 10 has a generally elongated body 12 of an appropriate length that extends to a distal end 13. Distal end 13 terminates at distal tip 14. Distal tip 14 is structured for penetrating, e.g., the outer skin of the patient, an occlusion, a body wall, an organ, or other bodily structure positioned along a path to a target site. Needle 10 has a beveled opening 16 that leads to a lumen (not shown) extending through the elongated body 12. As described herein, a beveled opening refers to an opening that is angled, or inclined, with regard to the main axis of the medical device at an angle other than a right angle. Needle 10 may also include an outer sheath 11 or like structure that extends to, or beyond, the proximal end (not shown) of the needle.

Prior art needle 10 includes an echogenic structure at its distal end. In this example, the echogenic structure comprises a pattern of dimpling 20 extending circumferentially around the distal end of the needle. It is known in the art to provide certain echogenic patterns along a medical device, such as a needle, and the pattern shown in Fig. 1 is one example of a known pattern. This and similar patterns are provided on needles commercially available from Cook Medical Technologies, LLC, Bloomington, IN, and referred to as ECHOTIP® needles. Other echogenic patterns, as well as devices to which such patterns have been applied, are disclosed in U.S. Pat. No. 4,869,259, and U.S. Pat. Publ. Nos. 2006/0247530, 2008/0097213, and 2011/0046619.

The known echogenic patterns, such as the pattern illustrated in the prior art needle of Fig. 1, have generally proven effective in enabling the ultrasound technician to locate, and track, the axial position of the distal tip of the needle within the body of the patient. However, this echogenic pattern is generally visible on the monitor as a brightened portion of the ultrasound image. The image is not provided in sufficient detail to enable the medical professional to determine the alignment, or rotational orientation, of the beveled opening of the needle.

It would be advantageous to have the capability of locating features of a medical device within the body of the patient with greater precision than available with prior art devices, such as the needle shown in Fig. 1. For example, it would be beneficial if the medical professional could determine the rotational orientation of the bevel of the needle. In this way, the professional could readily distinguish the position of the tip 14 of the bevel from the bevel heel 18. Armed with this knowledge, the professional could thereby ensure that the needle is oriented for insertion such that the tip enters the target structure first.

Without the ability to distinguish the rotational orientation of the needle as described, the medical professional may attempt to lead into the target site with the bevel heel side of the needle, instead of with the tip side. In this event, and particularly in those instances when entry is attempted at an angle to the vessel or other target structure, the stick may be unsuccessful, as the needle may deflect off the vessel or structure. When this occurs, the professional must rotate the needle in an attempt to lead with the needle tip 14. However, due to the lack of visibility on ultrasound, and in particular, an inability to distinguish the bevel heel from the distal tip on the ultrasound monitor, such rotation includes a certain element of trial and error. If sufficient rotation is not achieved, a second, or even a third attempt could also be unsuccessful. Any unsuccessful attempts add unnecessary time and effort to the procedure. Additionally, such unsuccessful attempts at entry may cause additional trauma to the patient. Even when entry is made, the professional can still not generally be certain of the exact orientation of the opening.

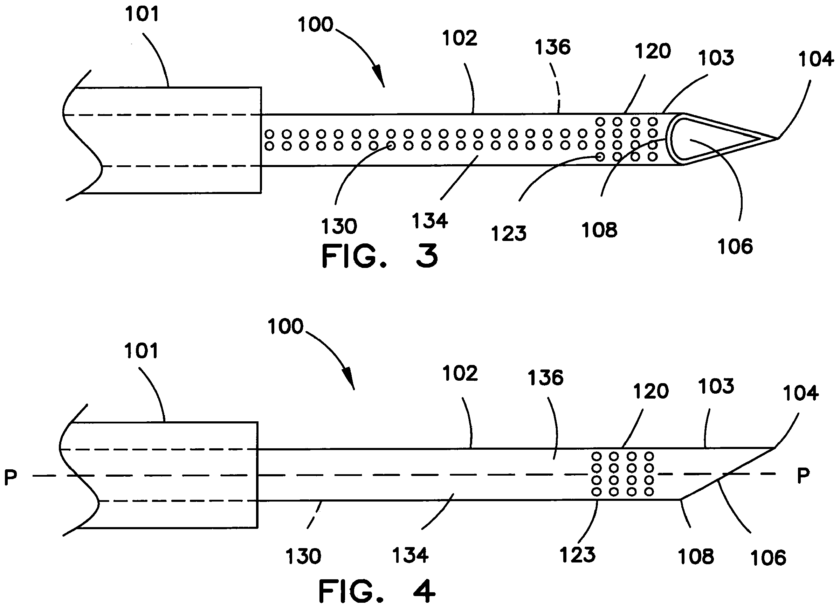

Fig. 3 illustrates a side view of the distal portion of an echogenic medical device according to an embodiment of the present invention. Fig. 4 is another side view of the distal portion of the device rotated 90 degrees from the orientation shown in Fig. 3. In the example of Figs. 3 and 4, the echogenic medical device comprises an echogenic needle 100. As illustrated, needle 100 has some similarities to prior art needle 10 shown in Figs. 1 and 2. For example, needle 100 has a generally elongated body 102 of an appropriate length, and extends to a beveled opening 106 at distal end 103. Beveled opening 106 is bordered at the distal end by distal tip 104, and at the proximal end by heel 108. As with the prior art needle, distal tip 104 is structured for penetrating, e.g., the outer skin of the patient, an occlusion, a body wall, an organ, or other bodily structure positioned along a path to the target site. Needle 100 may also include an outer sheath 101, or like structure, that extends to, or beyond, the proximal end (not shown) of the needle, as well known in the art.

In this example, needle 100 includes a circumferential pattern of dimpling 120 formed at the distal end. This circumferential pattern of dimpling may be similar to dimpling pattern 20 that is shown on prior art needle 10. As stated above with regard to pattern 20 of Fig. 1, dimpling pattern 120 is visualized on the ultrasound monitor as a brightened portion that informs the operator of the general position of the distal portion of the needle. Dimpling pattern 20 on the prior art device does not inform the operator of the degree of rotation of the needle. As a result, the respective positions of distal tip 14 and bevel heel 18 cannot generally be distinguished in the prior art needle with sufficient clarity to ensure that the distal tip 14 is initially inserted into the target structure.

In the example shown in Fig. 3, the position of the bevel heel 108 may be readily distinguished from the distal tip 104. Unlike prior art needle 10 of Figs. 1 and 2, needle 100 includes a second echogenic feature. In Fig. 3, this additional echogenic feature is an echogenic stripe 130. Echogenic stripe 130 comprises a pattern of dimples extending longitudinally along all or a portion of the length of one longitudinal side 134, or half, of needle 100. In Fig. 4 an imaginary plane, identified on the figure as P, extends along the longitudinal axis of needle 100 to distinguish longitudinal side 134 of the needle from the other longitudinal side 136. As illustrated in Fig. 3, echogenic stripe 130 comprises a pattern of dimples, two to a row, extending in a proximal direction along needle longitudinal side 134, from the most proximal circumferential row 123 of dimples 120. In this example, stripe 130 is oriented such that it commences proximal of heel 108 of beveled needle opening 106.

By providing echogenic stripe 130 along a discrete longitudinal side of needle 100, in this case on the longitudinal side 134 of elongated body 102 that includes heel 108, the operator can readily distinguish one longitudinal side, or half, of the needle from the other on the ultrasound monitor. As a result, the operator is therefore able to readily determine the location of the heel. Armed with this knowledge, the operator can also readily determine the position, or more importantly, the rotational orientation of needle tip 104. The operator can then readily determine from the image on the screen whether tip 104 must be rotated in order to lead into the vessel or other structure to be penetrated by needle tip 104. If the needle must be rotated in order to maneuver tip 104 into position for initial entry, the image provided by the echogenic stripe enables the operator to determine when sufficient rotation has been carried out such that the tip is properly positioned for initial entry.

Although the respective echogenic stripe 130 and circumferential pattern 120 have been described for simplicity as comprising a series of dimples, the use if this terminology is not meant to limit the echogenic feature to a particular geometric or structural configuration. Rather, those skilled in the art are aware that echogenicity may be imparted to a substrate in other ways. Thus, for example, the surface of the device may be constructed or treated in a manner such that a textured, patterned, indented, angled, or otherwise irregular surface may be formed thereon. As stated above, the dimpling may include dimples, divots, knurls, ridges, nubs, and like structures and configurations that are capable of enhancing the echogenicity as compared to a smooth or untreated surface for an otherwise similarly-sized/shaped object.

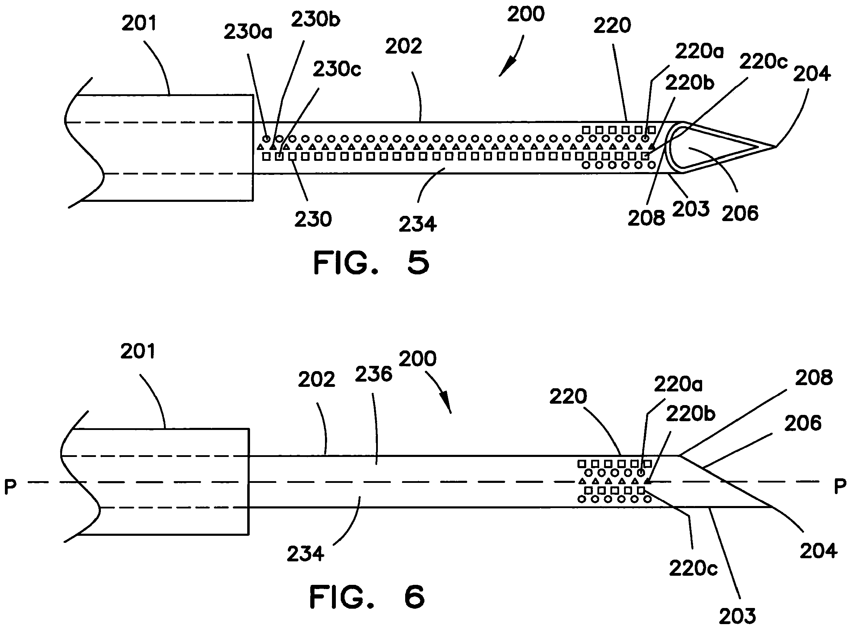

Figs. 5 and 6 illustrate another example of an echogenic medical device. In this example, echogenic needle 200 includes a generally elongated body 202 that extends to beveled opening 206 at distal end 203. Beveled opening 206 is bordered at the distal end by distal tip 204 and at the proximal end by heel 208. An outer sheath 201 may be provided as before.

As in the previous example, needle 200 includes a circumferential dimpling pattern 220 and an echogenic longitudinal stripe pattern 230. Longitudinal stripe pattern 230 may be provided along longitudinal side, or half, 234 of elongated body 202. One or both of patterns 220, 230 may comprise a plurality of geometrically-shaped echogenic elements. In this example, dimpling pattern 220 comprises alternating rows of echogenic dimples having different geometric shapes, and extending around the circumference of distal end 203. The alternating rows may comprise a sequential arrangement comprising a row 220a of generally circular dimples, a row 220b of generally triangular dimples, and a row 220c of generally square dimples. Similarly, longitudinal stripe pattern 230 may comprise respective longitudinal rows of generally circular dimples 230a, generally triangular dimples 230b, and generally square dimples 230c along longitudinal side 234. In this manner longitudinal side 234 may be distinguished from longitudinal side 236.

By providing respective echogenic regions 220, 230 formed of sequential rows of echogenic elements of various geometrical configurations, a suitable ultrasound image may be achieved from a wider range of insertion angles of the needle when compared to an image resulting from a single configuration.

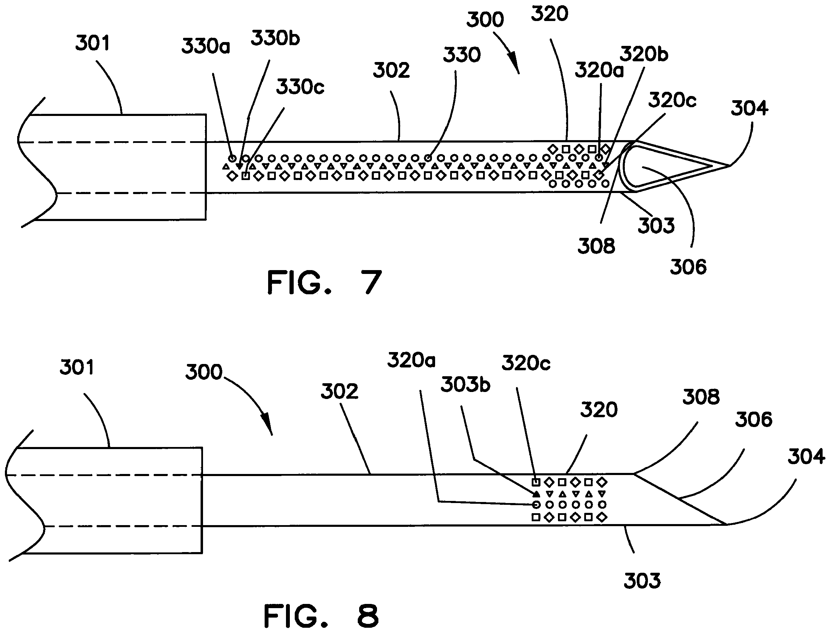

Figs. 7 and 8 illustrate another example of an echogenic medical device. In this example, echogenic needle 300 includes a generally elongated body 302 that extends to beveled opening 306 at distal end 303. Beveled opening 306 is bordered at the distal end by distal tip 304 and at the proximal end by heel 308. An outer sheath 301 may be provided to receive the proximal end of the needle body, as before.

As in the previous examples, needle 300 includes a circumferential dimpling pattern 320 and an echogenic stripe pattern 330. As in the example of Figs. 5 and 6, one or both of patterns 320, 330 may comprise alternating rows of echogenic dimples having geometric shapes that differ from the shapes of the elements in another row. Unlike the previous example, at least some of the echogenic elements in any particular row may be rotated about their individual axes. Thus, as shown, dimpling pattern 320 may comprise alternating rows of echogenic dimples comprising a row 320a of generally circular dimples, a row 320b of generally triangular dimples, and a row 320c of generally square dimples. Similarly, longitudinal stripe pattern 330 may comprise respective longitudinal rows of generally circular dimples 330a, generally triangular dimples 330b, and generally square dimples 330c.

Rotating at least some of the echogenic elements around their respective axes as described enhances the echogenic signal from a respective row of the elements when compared to the same row without such rotation of the elements, thereby enhancing the visibility of stripe pattern 330.

Although the echogenic elements 120, 130, 220, 230, 320, 330 previously shown and described are either generally circular, generally triangular, or generally square, these are only examples of possible geometric configurations of the echogenic elements. Those skilled in the art will appreciate that other geometric shapes and configurations, such as hexagonal, pyramidal, etc., may be substituted for the shapes of the dimples shown and described, as long as the geometric shapes and configurations are capable of providing a suitable signal for ultrasound imaging.

Similarly, although each of the rows in the examples shown and described includes echogenic elements having the same geometric configuration, this is not required in all instances. Therefore, it is permissible to include dimples of a plurality of geometric configurations in a single row, or in each row, at least some of which may be rotated about their respective axes when compared to other elements in the row, as described above.

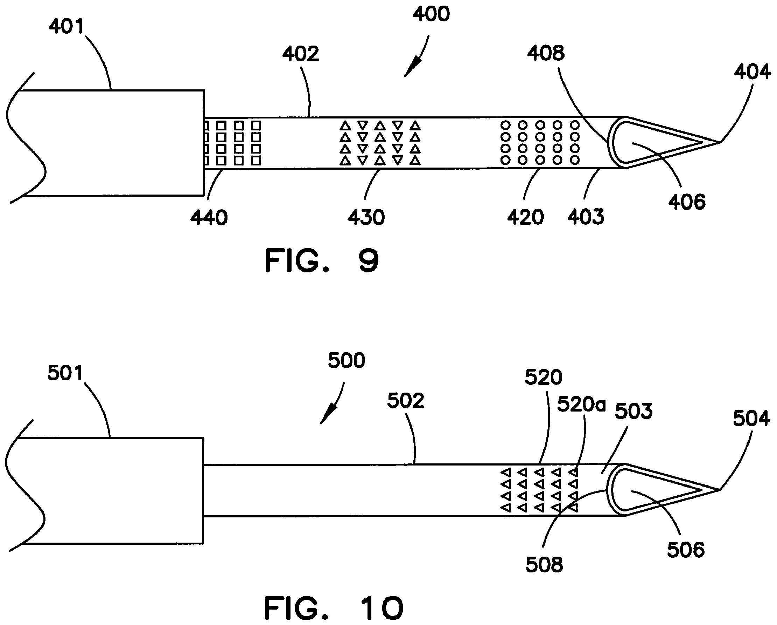

Fig. 9 illustrates another example of an echogenic medical device. In this example, echogenic needle 400 includes a generally elongated body 402 that extends to beveled opening 406 at distal end 403. Beveled opening 406 is bordered at the distal end by distal tip 404 and at the proximal end by heel 408, as in the previous examples. An outer sheath 401 may be provided as before.

Needle 400 includes a first circumferential dimpling pattern 420 as before, and includes one or more sets of additional dimpling patterns. In this example, needle 400 includes second and third dimpling patterns 430, 440, respectively. Dimpling patterns 420, 430, 440 may be formed of echogenic elements of a type described above. In this example, the echogenic elements of the first circumferential pattern 420 are generally circular. The echogenic elements of the second circumferential pattern 430 are generally triangular. The echogenic elements of the third circumferential pattern are generally square. If desired, at least some of the echogenic elements may be rotated in any fashion about their axes. See, e.g., generally triangular elements 430 in the example shown. Those skilled in the art will appreciate that the specific geometric configurations of the echogenic elements are not restricted to the elements shown in this example, and may be varied as desired. Providing echogenic bands of different configurations spaced longitudinally along the length of the needle allows the operator to better determine penetration depth, and provides a scale along the shaft of the needle composed of various shapes and angles.

Fig. 10 illustrates still another example of an echogenic medical device. Echogenic needle 500 includes a generally elongated body 502 that extends to beveled opening 506 at distal end 503. Beveled opening 506 is bordered at the distal end by distal tip 504 and at the proximal end by heel 508. An outer sheath 501 may be provided as before. A circumferential pattern of dimpling 520 is formed at the distal end. In this example, dimples 520 extend inwardly into the surface of the needle in a manner to comprise one or more wall angles θ₁, θ₂, etc. In the example shown, generally-triangular dimples extend inwardly into the matrix of the needle in a generally pyramidal configuration to create the wall angles θ₁ and θ₂.

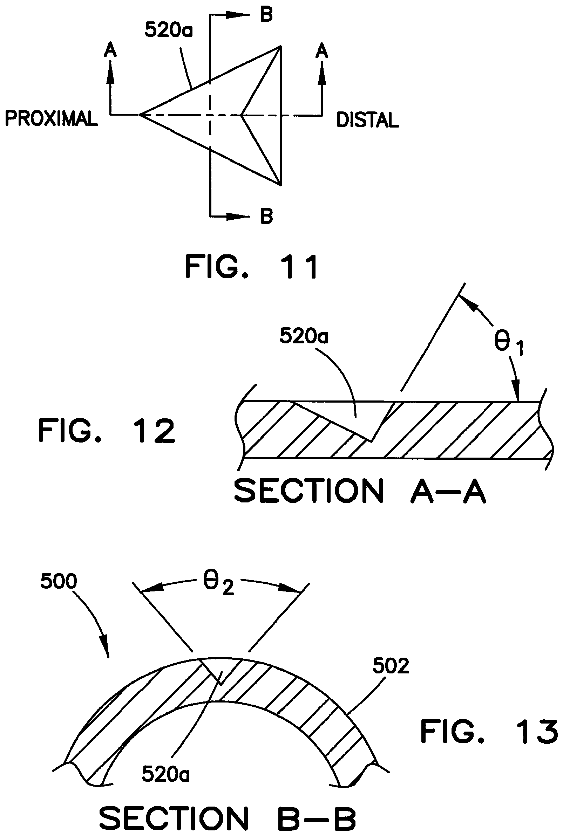

Fig. 11 is an enlarged view of one of dimples 520, in this case dimple 520a. Lines A-A and B-B are provided to illustrate the orientation of angles θ₁ and θ₂. Fig. 12 is a tangential sectional view of needle 500 at dimple 520a. This tangential cross-section view is taken along A-A of Fig. 11, and illustrates angle θ₁. Fig. 13 is an axial sectional view along the circumference of needle 500 at dimple 520a. This axial sectional view is taken along B-B of Fig. 11, and illustrates angle θ₂. Those skilled in the art may adjust the configuration of the dimples, and the respective wall angles, as desired to enhance or otherwise modify echogenicity.

In addition to the dimples and related structures that may impart echogenicity to a medical device as described, echogenicity may be also imparted to the device in a manner other than by forming the echogenic elements into or onto the surface of the device in a manner described above. For example, more, or fewer, rows of dimples, etc., may be applied to form longitudinal stripes, such as stripes 130, 230, 330, and the other echogenic structures as shown and described hereinabove. In addition, the rows need not necessarily be adjacent as shown in Figs. 3, 5, and 7, and instead can be spaced or otherwise positioned in a manner such that a discernable and/or distinguishable echogenic pattern may be observed. Further, instead of longitudinal rows, other patterns can be substituted, such as spiral rows, broken lines, etc., along the longitudinal side of the needle.

Those skilled in the art will appreciate that since an objective is to distinguish an amount of rotation of the device, other patterns capable of providing such orientation may be substituted. Preferably, however, all or most of the pattern will extend along a particular longitudinal side of the device. This arrangement provides a very favorable frame of reference, so that the rotational orientation of the medical device can be readily determined. Those skilled in the art will appreciate that any irregularities or other modification of the substrate should be carried out in a manner that does not adversely affect the mechanical properties of the substrate in any material fashion.

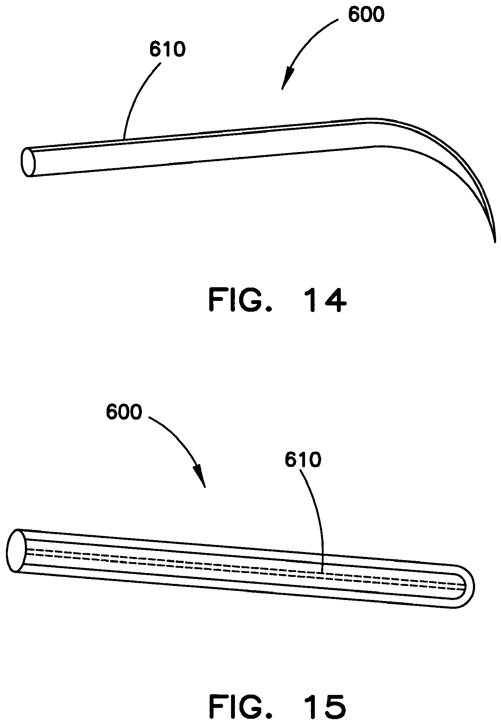

Fig. 14 is another example of an echogenic medical device. Fig. 14 illustrates a medical device, such as curved catheter 600, having an echogenic ribbon 610 applied along a length of the catheter. In Fig. 14, the echogenic ribbon is disposed along an outer surface of the curved portion. Fig. 15 illustrates the curved catheter rotated 90 degrees from the view of Fig. 14 so that the top surface of the curved catheter can be observed.

In this embodiment, rather than deforming the surface of the catheter to form the series of irregularities as described, one or more lengths of echogenic ribbon 610 may be provided along all, or a portion, of the length of catheter 600. Ribbon 610 may be formed of the same or a similar composition as catheter 600, such as a metal or a metal alloy, and deformations (e.g., dimples) are disposed along the surface of the ribbon. The deformations may be formed in the same manner as the deformations on the structures previously described, and may be dispersed along the surface of ribbon 610 in a manner such that an operator may discern the orientation of the catheter. Although catheter 600 and ribbon 610 have been described in this example as being formed of the same or a similar material, those skilled in the art will appreciate that this need not be the case, as long as suitable means (e.g., an adhesive or bonding) are provided for securing the ribbon along the surface of the catheter.

Although ribbon 610 is shown in Figs. 14 and 15 as a continuous ribbon extending along the length of catheter 600, other configurations are possible. For example, if desired, multiple shorter ribbons or similar structures can be applied to, or wrapped around, catheter 600. This arrangement may also provide additional flexibility to the echogenic portion of the feeding tube. Alternatively or additionally, echogenicity may be imparted by surface deformation or irregularity, or by modifying the matrix of the substrate. As still another alternative, ribbon 610 can be embedded into the wall of catheter 600, and a plastic can be extruded or otherwise applied over the ribbon and the catheter. Those skilled in the art will appreciate that although a curved medical device 600 is illustrated in the example, this is merely one example of a medical device that may be modified by application of ribbon 610, and that a modification of devices of other configurations, such as straight, helical, etc. is also contemplated.

In addition to the foregoing, there are additional ways of providing echogenicity to a substrate of a type that will result in enhanced scatter and/or reflectance of ultrasound signals, and that may be substituted for the surface techniques described above. For example, an echogenic coating can be applied to a designated length of the substrate, such as the length of longitudinal echogenic stripes 130, 230, 330. Suitable echogenic coatings are described in, for example, U.S. Patent Nos. 6,506,156 and 6,106,473. As yet another variation, instead of surface modification or utilizing a separate echogenic ribbon, stripe, coating, etc., the substrate may be formed to have an echogenic material incorporated into all or any designated portion of its matrix. Thus, for example, a known material for imparting echogenicity, such as glass spheres, echogenic metal or alloys (e.g., tungsten), etc., may be incorporated into the matrix during formation of the substrate, e.g., into a polymer matrix during substrate formation. Preferably, the materials (e.g., the glass spheres) will only be incorporated into the distal portion or other specifically designated portion of the substrate, and will be incorporated in a manner such that a distinct echogenic pattern is provided along the designated substrate portion, such as longitudinal side 134 (Figs. 3 and 4). Alternatively or additionally, a portion (e.g., the distal portion) of the substrate can be formed to have the desired echogenic pattern, and this portion can then be affixed (e.g., via heat bonding, adhesion, etc.) to another portion (e.g., the proximal portion or an intermediate portion) of the substrate. Once this substrate is formed, there would generally be no further need to add other irregularities, materials, etc., to enhance echogenicity, as sufficient echogenicity for visualization under ultrasound is provided by the matrix materials.

It is therefore intended that the foregoing detailed description be regarded as illustrative rather than limiting, and that it be understood that it is the following claims, including all equivalents, that are intended to define the scope of this invention.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. An echogenic needle (100) configured for insertion into the body of a patient and ultrasound-guided movement therein to an interior target site, comprising:
a shaft having a proximal portion, a distal portion extending to a distal end (103), first and second generally opposing longitudinal sides extending along said proximal and distal portions, and a passageway extending therethrough, said distal end (103) defining a beveled opening (106) communicating with said passageway, said beveled opening (106) extending between a distal tip portion disposed along said first longitudinal side (136) and a heel portion disposed along said second longitudinal side (134); **characterized by** a first echogenic region extending circumferentially around the shaft at said distal portion, said first echogenic region structured for providing a signal visible along substantially the entire circumference of the shaft under ultrasound visualization; and
a second echogenic region proximal of said first echogenic region, said second echogenic region extending along a length of said second longitudinal side (134) and substantially aligned with said heel portion, said second echogenic region structured and arranged for providing a generally linear signal visible under ultrasound examination along said second longitudinal side (134), and substantially not visible along said first longitudinal side (136).

2. The echogenic needle (100) of claim 1, wherein at least said second echogenic region comprises a plurality of echogenic members, said second echogenic members comprising a plurality of geometric configurations.

3. The echogenic needle (100) of any one of the preceding claims, wherein said echogenic members of said second echogenic region are aligned in respective rows, said members in a first row having a first geometric configuration and said members in a second row having a second geometric configuration, different from said first configuration; wherein said second echogenic region preferably comprises a third row of echogenic members, said members in said third row having a different configuration than said members in said first and second rows.

4. The echogenic needle (100) of claim 3, wherein each of said echogenic members has an axis, and wherein at least some of said echogenic members have a different rotational alignment along said axis with reference to other echogenic members in said row.

5. The echogenic needle (100) of any one of the preceding claims, wherein said second echogenic region comprises a plurality of echogenic members, at least some of said echogenic members comprising a geometric configuration extending into said shaft at a plurality of angles.

6. The echogenic needle (100) of any one of the preceding claims, wherein at least said first echogenic region comprises a plurality of echogenic members, said first echogenic members comprising a plurality of geometric configurations disposed along said distal portion, at least some of said geometric configurations extending into a matrix of said shaft and defining at least two wall angles θ₁, θ₂, each of said wall angles configured and positioned to enhance an echogenicity of said geometric configuration under ultrasound visualization.

7. The echogenic needle (100) of any one of the preceding claims, wherein at least said first echogenic region comprises a plurality of echogenic members, said first echogenic members comprising a plurality of geometric configurations.

8. The echogenic needle (100) of any one of the preceding claims, wherein said echogenic members of said first echogenic region are aligned in respective rows, said members in a first row having a first geometric configuration and said members in a second row having a second geometric configuration, different from said first configuration; wherein said first echogenic region preferably comprises a third row of echogenic members, said members in said third row having a different configuration than said members in said first and second rows.

9. The echogenic needle (100) of claim 8, wherein each of said first echogenic members has an axis, and wherein at least some of said echogenic members have a different rotational alignment along said axis with reference to other echogenic members in said row.

10. The echogenic needle (100) of any one of the preceding claims, wherein said first echogenic region comprises a plurality of echogenic members at least some of said echogenic members comprising a geometric configuration extending into said shaft at a plurality of angles.

11. The echogenic needle (100) of claim 1, wherein said first echogenic region comprises a plurality of first echogenic members and said second echogenic region comprises a plurality of second echogenic members, said first echogenic members comprising a plurality of geometric configurations and said second echogenic members comprising a plurality of geometric configurations.

12. The echogenic needle (100) of claim 11, wherein said first echogenic members of said first echogenic region are aligned in respective rows, said first echogenic members in a first row having a first geometric configuration and said first echogenic members in a second row having a second geometric configuration, different from said first configuration, and said second echogenic members of said second echogenic region are aligned in respective rows, said second echogenic members in a first row having a first geometric configuration and said second echogenic members in a second row having a second geometric configuration, different from said first configuration.

13. The echogenic needle (100) of claim 12, wherein said first echogenic region comprising a third row of first echogenic members, said first echogenic members in said third row having a different configuration than said first echogenic members in said first and second rows, and said second echogenic region comprising a third row of second echogenic members, said second echogenic members in said third row having a different configuration than said second echogenic members in said first and second rows.

14. The echogenic needle (100) of claim 12 or 13, wherein each of said first echogenic members has an axis, and wherein at least some of said first echogenic members have a different rotational alignment along said axis with reference to other first echogenic members in said row, and each of said second echogenic members has an axis, and wherein at least some of said second echogenic members have a different rotational alignment along said axis with reference to other second echogenic members in said row.

15. The echogenic needle (100) of claim 1, wherein said first echogenic region comprises a plurality of first echogenic members at least some of said first echogenic members comprising a geometric configuration extending into said shaft at a plurality of angles, and said second echogenic region comprises a plurality of second echogenic members at least some of said second echogenic members comprising a geometric configuration extending into said shaft at a plurality of angles.

## Patentansprüche

1. Echogene Nadel (100), die zur Einführung in den Körper eines Patienten und für eine unter Ultraschall geführte Bewegung darin bis zu einer inneren Zielstelle ausgelegt ist, umfassend:
einen Schaft mit einem proximalen Abschnitt, einem sich bis zu einem distalen Ende (103) erstreckenden distalen Abschnitt, ersten und zweiten allgemein gegenüberliegenden Längsseiten, die sich entlang der proximalen und distalen Abschnitte erstrecken, und einem sich dort hindurch erstreckenden Durchgang, wobei das distale Ende (103) eine abgeschrägte Öffnung (106) definiert, die mit dem Durchgang in Verbindung steht, wobei sich die abgeschrägte Öffnung (106) zwischen einem distalen Spitzenabschnitt, der entlang der ersten Längsseite (136) angeordnet ist, und einem Fersenabschnitt, der entlang der zweiten Längsseite (134) angeordnet ist, erstreckt;
**gekennzeichnet durch**
eine erste echogene Region, die sich um den Umfang des Schafts am distalen Abschnitt erstreckt, wobei die erste echogene Region zur Bereitstellung eines Signals strukturiert ist, das im Wesentlichen entlang des gesamten Umfangs des Schafts unter Ultraschallvisualisierung sichtbar ist; und
eine zweite echogene Region proximal von der ersten echogenen Region, wobei sich die zweite echogene Region entlang einer Länge der zweiten Längsseite (134) erstreckt und im Wesentlichen mit dem Fersenabschnitt ausgerichtet ist, wobei die zweite echogene Region zur Bereitstellung eines allgemein linearen Signals strukturiert und angeordnet ist, das bei einer Ultraschalluntersuchung entlang der zweiten Längsseite (134) sichtbar ist und entlang der ersten Längsseite (136) im Wesentlichen nicht sichtbar ist.

2. Echogene Nadel (100) nach Anspruch 1, wobei zumindest die zweite echogene Region eine Vielzahl von echogenen Elementen umfasst, wobei die zweiten echogenen Elemente eine Vielzahl von geometrischen Konfigurationen umfassen.

3. Echogene Nadel (100) nach einem der vorhergehenden Ansprüche, wobei die echogenen Elemente der zweiten echogenen Region in jeweiligen Reihen ausgerichtet sind, wobei die Elemente in einer ersten Reihe eine erste geometrische Konfiguration aufweisen und die Elemente in einer zweiten Reihe eine sich von der ersten Konfiguration unterscheidende zweite geometrische Konfiguration aufweisen; wobei die zweite echogene Region vorzugsweise eine dritte Reihe von echogenen Elementen umfasst, wobei die Elemente in der dritten Reihe eine andere Konfiguration als die Elemente in der ersten und zweiten Reihe aufweisen.

4. Echogene Nadel (100) nach Anspruch 3, wobei jedes der echogenen Elemente eine Achse aufweist und wobei zumindest einige der echogenen Elemente bezüglich anderer echogener Elemente in der Reihe eine andere Drehausrichtung entlang der Achse aufweisen.

5. Echogene Nadel (100) nach einem der vorhergehenden Ansprüche, wobei die zweite echogene Region eine Vielzahl von echogenen Elementen umfasst, wobei zumindest einige der echogenen Elemente eine geometrische Konfiguration umfassen, die sich in einer Vielzahl von Winkeln in den Schaft erstreckt.

6. Echogene Nadel (100) nach einem der vorhergehenden Ansprüche, wobei zumindest die erste echogene Region eine Vielzahl von echogenen Elementen umfasst, wobei die ersten echogenen Elemente eine Vielzahl von geometrischen Konfigurationen umfassen, die entlang des distalen Abschnitts angeordnet sind, wobei sich zumindest einige der geometrischen Konfigurationen in eine Matrix des Schafts erstrecken und mindestens zwei Wandwinkel θ₁, θ₂ definieren, wobei jeder dieser Wandwinkel zur Erhöhung einer Echogenität der geometrischen Konfiguration unter Ultraschallvisualisierung ausgelegt und positioniert ist.

7. Echogene Nadel (100) nach einem der vorhergehenden Ansprüche, wobei zumindest die erste echogene Region eine Vielzahl von echogenen Elementen umfasst, wobei die ersten echogenen Elemente eine Vielzahl von geometrischen Konfigurationen umfassen.

8. Echogene Nadel (100) nach einem der vorhergehenden Ansprüche, wobei die echogenen Elemente der ersten echogenen Region in jeweiligen Reihen ausgerichtet sind, wobei die Elemente in einer ersten Reihe eine erste geometrische Konfiguration aufweisen und die Elemente in einer zweiten Reihe eine sich von der ersten Konfiguration unterscheidende zweite geometrische Konfiguration aufweisen; wobei die erste echogene Region vorzugsweise eine dritte Reihe von echogenen Elementen umfasst, wobei die Elemente in der dritten Reihe eine andere Konfiguration als die Elemente in der ersten und zweiten Reihe aufweisen.

9. Echogene Nadel (100) nach Anspruch 8, wobei jedes der ersten echogenen Elemente eine Achse aufweist und wobei zumindest einige der echogenen Elemente bezüglich anderer echogener Elemente in der Reihe eine andere Drehausrichtung entlang der Achse aufweisen.

10. Echogene Nadel (100) nach einem der vorhergehenden Ansprüche, wobei die erste echogene Region eine Vielzahl von echogenen Elementen umfasst, wobei zumindest einige der echogenen Elemente eine geometrische Konfiguration umfassen, die sich in einer Vielzahl von Winkeln in den Schaft erstreckt.

11. Echogene Nadel (100) nach Anspruch 1, wobei die erste echogene Region eine Vielzahl von ersten echogenen Elementen umfasst und die zweite echogene Region eine Vielzahl von zweiten echogenen Elementen umfasst, wobei die ersten echogenen Elemente eine Vielzahl von geometrischen Konfigurationen umfassen und die zweiten echogenen Elemente eine Vielzahl von geometrischen Konfigurationen umfassen.

12. Echogene Nadel (100) nach Anspruch 11, wobei die ersten echogenen Elemente der ersten echogenen Region in jeweiligen Reihen ausgerichtet sind, wobei die ersten echogenen Elemente in einer ersten Reihe eine erste geometrische Konfiguration aufweisen und die ersten echogenen Elemente in einer zweiten Reihe eine sich von der ersten Konfiguration unterscheidende zweite geometrische Konfiguration aufweisen und die zweiten echogenen Elemente der zweiten echogenen Region in jeweiligen Reihen ausgerichtet sind, wobei die zweiten echogenen Elemente in einer ersten Reihe eine erste geometrische Konfiguration aufweisen und die zweiten echogenen Elemente in einer zweiten Reihe eine sich von der ersten Konfiguration unterscheidende zweite geometrische Konfiguration aufweisen.

13. Echogene Nadel (100) nach Anspruch 12, wobei die erste echogene Region eine dritte Reihe von ersten echogenen Elementen umfasst, wobei die ersten echogenen Elemente in der dritten Reihe eine andere Konfiguration als die ersten echogenen Elemente in der ersten und zweiten Reihe aufweisen und die zweite echogene Region eine dritte Reihe von zweiten echogenen Elementen umfasst, wobei die zweiten echogenen Elemente in der dritten Reihe eine andere Konfiguration als die zweiten echogenen Elemente in der ersten und zweiten Reihe aufweisen.

14. Echogene Nadel (100) nach Anspruch 12 oder 13, wobei jedes der ersten echogenen Elemente eine Achse aufweist und wobei zumindest einige der ersten echogenen Elemente bezüglich anderer erster echogener Elemente in der Reihe eine andere Drehausrichtung entlang der Achse aufweisen, und wobei jedes der zweiten echogenen Elemente eine Achse aufweist und wobei zumindest einige der zweiten echogenen Elemente bezüglich anderer zweiter echogener Elemente in der Reihe eine andere Drehausrichtung entlang der Achse aufweisen.

15. Echogene Nadel (100) nach Anspruch 1, wobei die erste echogene Region eine Vielzahl von ersten echogenen Elementen umfasst, wobei zumindest einige der ersten echogenen Elemente eine geometrische Konfiguration umfassen, die sich in einer Vielzahl von Winkeln in den Schaft erstreckt, und wobei die zweite echogene Region eine Vielzahl von zweiten echogenen Elementen umfasst, wobei zumindest einige der zweiten echogenen Elemente eine geometrische Konfiguration umfassen, die sich in einer Vielzahl von Winkeln in den Schaft erstreckt.

## Revendications

1. Aiguille échogène (100) configurée en vue d'une insertion dans l'organisme d'un patient et d'un déplacement guidé par ultrasons dans celui-ci jusqu'à un site cible intérieur, comprenant :
une tige comportant une partie proximale, une partie distale s'étendant jusqu'à une extrémité distale (103), des premier et second côtés longitudinaux globalement opposés s'étendant le long desdites parties proximale et distale, et un passage s'étendant à travers elle, ladite extrémité distale (103) définissant une ouverture biseautée (106) communiquant avec ledit passage, ladite ouverture biseautée (106) s'étendant entre une partie de pointe distale disposée le long dudit premier côté longitudinal (136) et une partie de base disposée le long dudit second côté longitudinal (134) ;
**caractérisée par** une première région échogène s'étendant de manière circonférentielle autour de la tige au niveau de ladite partie distale, ladite première région échogène étant structurée de façon à produire un signal visible le long d'essentiellement la totalité de la circonférence de la tige dans le cadre d'une visualisation par ultrasons ; et
une seconde région échogène, proximale vis-à-vis de ladite première région échogène, ladite seconde région échogène s'étendant le long d'une longueur dudit second côté longitudinal (134) et étant essentiellement alignée sur ladite partie de base, ladite seconde région échogène étant structurée et conçue de façon à produire un signal globalement linéaire visible dans le cadre d'un examen par ultrasons le long dudit second côté longitudinal (134), et essentiellement non visible le long dudit premier côté longitudinal (136).

2. Aiguille échogène (100) selon la revendication 1, dans laquelle au moins ladite seconde région échogène comprend une pluralité d'éléments échogènes, lesdits seconds éléments échogènes comprenant une pluralité de configurations géométriques.

3. Aiguille échogène (100) selon l'une quelconque des revendications précédentes, dans laquelle lesdits éléments échogènes de ladite seconde région échogène sont alignés en rangées respectives, lesdits éléments dans une première rangée présentant une première configuration géométrique et lesdits éléments dans une deuxième rangée présentant une deuxième configuration géométrique, différente de ladite première configuration; dans laquelle ladite seconde région échogène comprend, de préférence, une troisième rangée d'éléments échogènes, lesdits éléments dans ladite troisième rangée présentant une configuration différente de celle desdits éléments dans lesdites première et deuxième rangées.

4. Aiguille échogène (100) selon la revendication 3, dans laquelle chacun desdits éléments échogènes présente un axe, et dans laquelle au moins certains desdits éléments échogènes présentent un alignement angulaire différent le long dudit axe par rapport à d'autres éléments échogènes dans ladite rangée.

5. Aiguille échogène (100) selon l'une quelconque des revendications précédentes, dans laquelle ladite seconde région échogène comprend une pluralité d'éléments échogènes, au moins certains desdits éléments échogènes comprenant une configuration géométrique s'étendant dans ladite tige à une pluralité d'angles.

6. Aiguille échogène (100) selon l'une quelconque des revendications précédentes, dans laquelle au moins ladite première région échogène comprend une pluralité d'éléments échogènes, lesdits premiers éléments échogènes comprenant une pluralité de configurations géométriques disposées le long de ladite partie distale, au moins certaines desdites configurations géométriques s'étendant dans une matrice de ladite tige et définissant au moins deux angles de paroi θ₁, θ₂, chacun desdits angles de paroi étant configuré et positionné de façon à augmenter une échogénicité de ladite configuration géométrique dans le cadre d'une visualisation par ultrasons.

7. Aiguille échogène (100) selon l'une quelconque des revendications précédentes, dans laquelle au moins ladite première région échogène comprend une pluralité d'éléments échogènes, lesdits premiers éléments échogènes comprenant une pluralité de configurations géométriques.

8. Aiguille échogène (100) selon l'une quelconque des revendications précédentes, dans laquelle lesdits éléments échogènes de ladite première région échogène sont alignés en rangées respectives, lesdits éléments dans une première rangée présentant une première configuration géométrique et lesdits éléments dans une deuxième rangée présentant une deuxième configuration géométrique, différente de ladite première configuration; dans laquelle ladite première région échogène comprend, de préférence, une troisième rangée d'éléments échogènes, lesdits éléments dans ladite troisième rangée présentant une configuration différente de celle desdits éléments dans lesdites première et deuxième rangées.

9. Aiguille échogène (100) selon la revendication 8, dans laquelle chacun desdits premiers éléments échogènes présente un axe, et dans laquelle au moins certains desdits éléments échogènes présentent un alignement angulaire différent le long dudit axe par rapport à d'autres éléments échogènes dans ladite rangée.

10. Aiguille échogène (100) selon l'une quelconque des revendications précédentes, dans laquelle ladite première région échogène comprend une pluralité d'éléments échogènes, au moins certains desdits éléments échogènes comprenant une configuration géométrique s'étendant dans ladite tige à une pluralité d'angles.

11. Aiguille échogène (100) selon la revendication 1, dans laquelle ladite première région échogène comprend une pluralité de premiers éléments échogènes et ladite seconde région échogène comprend une pluralité de seconds éléments échogènes, lesdits premiers éléments échogènes comprenant une pluralité de configurations géométriques et lesdits seconds éléments échogènes comprenant une pluralité de configurations géométriques.

12. Aiguille échogène (100) selon la revendication 11, dans laquelle lesdits premiers éléments échogènes de ladite première région échogène sont alignés en rangées respectives, lesdits premiers éléments échogènes dans une première rangée présentant une première configuration géométrique et lesdits premiers éléments échogènes dans une deuxième rangée présentant une deuxième configuration géométrique, différente de ladite première configuration, et lesdits seconds éléments échogènes de ladite seconde région échogène sont alignés en rangées respectives, lesdits seconds éléments échogènes dans une première rangée présentant une première configuration géométrique et lesdits seconds éléments échogènes dans une deuxième rangée présentant une deuxième configuration géométrique, différente de ladite première configuration.

13. Aiguille échogène (100) selon la revendication 12, dans laquelle ladite première région échogène comprend une troisième rangée de premiers éléments échogènes, lesdits premiers éléments échogènes dans ladite troisième rangée présentant une configuration différente de celle desdits premiers éléments échogènes dans lesdites première et deuxième rangées, et ladite seconde région échogène comprend une troisième rangée de seconds éléments échogènes, lesdits seconds éléments échogènes dans ladite troisième rangée présentant une configuration différente de celle desdits seconds éléments échogènes dans lesdites première et deuxième rangées.

14. Aiguille échogène (100) selon la revendication 12 ou 13, dans laquelle chacun desdits premiers éléments échogènes présente un axe, et dans laquelle au moins certains desdits premiers éléments échogènes présentent un alignement angulaire différent le long dudit axe par rapport à d'autres premiers éléments échogènes dans ladite rangée, et chacun desdits seconds éléments échogènes présente un axe, et dans laquelle au moins certains desdits seconds éléments échogènes présentent un alignement angulaire différent le long dudit axe par rapport à d'autres seconds éléments échogènes dans ladite rangée.

15. Aiguille échogène (100) selon la revendication 1, dans laquelle ladite première région échogène comprend une pluralité de premiers éléments échogènes, au moins certains desdits premiers éléments échogènes comprenant une configuration géométrique s'étendant dans ladite tige à une pluralité d'angles, et ladite seconde région échogène comprend une pluralité de seconds éléments échogènes, au moins certains desdits seconds éléments échogènes comprenant une configuration géométrique s'étendant dans ladite tige à une pluralité d'angles.
